# EUROPEAN PATENT SPECIFICATION

(11) **EP 4 335 402 B1**
(45) Date of publication and mention of the grant of the patent: **04.11.2026**
(21) Application number: 23162028.7
(22) Date of filing: 15.03.2023
(51) Int. Cl.: A61B 34/10

(54) **METHOD FOR MODELLING AN ENDOPROTHESIS IMPLANTED IN A PORTION OF A PATIENT AORTA**
VERFAHREN ZUR MODELLIERUNG EINER IN EINEM TEIL EINER AORTA EINES PATIENTEN IMPLANTIERTEN ENDOPROTHESE
PROCÉDÉ DE MODÉLISATION D'UNE ENDOPROTHÈSE IMPLANTÉE DANS UNE PARTIE D'UNE AORTE DE PATIENT

(30) Priority: 08.09.2022 US 202263404700 P
(43) Date of publication of application: 13.03.2024
(73) Proprietor: Predisurge, 42000 Saint-Etienne (FR)
(72) Inventor: VERMUNT, Joris, 42000 SAINT-ETIENNE (FR); ALBERTINI, Jean-Noël, 42000 SAINT-ETIENNE (FR); PERRIN, David, 42000 SAINT-ETIENNE (FR)
(74) Representative: Icosa

(56) References cited:
- WO-A1-2018/073505
- US-A1- 2018 289 422
- US-A1- 2020 364 936

## Description

### FIELD OF INVENTION

The present invention relates to the field of numerical simulations of cardiovascular surgical interventions.

More specifically, the invention concerns a computer implementing method for modelling an endoprosthesis implanted in a portion of a patient aorta in order to improve surgery outcomes.

### BACKGROUND OF INVENTION

Cardiovascular surgical interventions are often followed by complications such as endoleaks, endoprosthesis migration, endoprosthesis infolding, endoprosthesis occlusion, thrombosis, and endoprosthesis kinks. In this context, there is room for improvement notably in the pre-operative phase of planification. The present invention provides a solution to allow evaluation of outcomes of the surgery beforehand and also personalization of the surgery.

Document US2018289422 A1 discloses a method and system for determining a measure of a risk of a patient developing cardiac conduction abnormalities as a result of transcatheter cardiac treatment. The method includes providing a patient-specific anatomical model representing cardiac region and an implant model representing a finite element representation of a cardiac implant. The method includes virtually placing said implant model into said patient-specific anatomical model. A measure of mechanical interaction between the implant model and the patient-specific anatomical model is determined and a measure of risk of the patient developing cardiac conduction abnormalities is determined on the basis of the determined mechanical interaction.

### SUMMARY

The presently claimed invention provides a computer-implemented method for simulating an interaction between an endoprosthesis dedicated to be implanted in a portion of a patient aorta and said portion of said patient aorta according to claim 1, a device comprising a processor according to claim 12, and a computer program product according to claim 13. Further developments of the herein claimed invention are described in the dependent claims. This disclosure relates to a computer-implemented method for simulating an interaction between an endoprosthesis dedicated to be implanted in a portion of a patient aorta and said portion of said patient aorta, so as to determine areas with risk of surgical complications, comprising the steps of:
- receiving 3D images of said portion of the patient aorta,
- reconstructing a deformable tridimensional digital model of said portion of the patient aorta based on said 3D images, the reconstructed deformable tridimensional digital model being referred to as aorta model,
- positioning a tridimensional digital model of said endoprosthesis at a planned location in the aorta model, said tridimensional model of said endoprosthesis and said aorta model forming together an implantation model,
- simulating said interaction based on a computation using said implantation model,
- determining, in the implantation model, based on results of said simulated interaction, at least one risk index of surgical complications for predefined areas.

For instance, one global risk index can be determined, for the whole endoprosthesis. In other examples, the predefined areas depend on the type of surgical complications. For instance, when the surgical complications is a type Ia endoleak, the defined areas are the proximal covered stents. In the present disclosure, by "endoprosthesis", it is meant a "stent-graft". When the surgical complication is a risk of migration, the defined area is the proximal uncovered stent. When the surgical complication is a risk of infolding or occlusion, the predefined area is the whole endoprosthesis. The predefined areas depend on the endoprosthesis manufacturer to be precise.

Thus, the method allows, by using patient-related data such as anatomical images and planification data such as a numerical model of an endoprosthesis planned to be implanted in the body of a patient, foreseeing potential areas of surgical complications. Meanwhile, the method allows personalizing a surgical intervention comprising an endoprosthesis implantation.

Advantageously, the surgical complications comprise at least one among endoleaks, endoprosthesis migration, endoprosthesis infolding, endoprosthesis occlusion, thrombosis, and endoprosthesis kinks.

In some embodiments, the computation involving said implantation model comprises a computation of a geometrical deformation of said tridimensional digital model of said endoprosthesis inside the aorta model.

In some embodiments, the computation of the geometrical deformation is performed by finite element analysis.

Advantageously, the 3D images are preoperative CT scan images. Thus, the method allows foreseeing surgical complications before a surgical intervention is carried out. This is advantageous, as current techniques are based on post-operative data. Therefore, the method allows improving the planification of a surgical intervention by rendering it possible testing different equipment to be used during the surgical intervention.

In some embodiments, the interaction is an endovascular repair of aortic aneurysms.

In some embodiments, the method further comprises, after determining the areas with risk of surgical complication, a step of determining, for each area with risk of surgical complication, a risk level.

In some embodiments, the step of determining a risk level comprises a computation involving an apposition index or an aspect ratio.

In some embodiments, each risk level is associated with a corresponding display pattern, the method further comprising a step of applying the corresponding display pattern to each area with risk of surgical complication.

Advantageously, the method further comprises a step of displaying the implantation model on a visualization unit with the areas with risk of surgical complication with the applied corresponding display pattern.

Another aspect of the invention relates to a method for planning an abdominal aortic aneurysm repair surgery using areas with risk of surgical complications determined by the method according to any of the preceding claims. As already mentioned, by using the method for simulating an interaction between an endoprosthesis and a portion of a patient aorta, it is possible to test different models of endoprosthesis and to select one that will minimize the risk of surgical complications.

Another aspect of the invention relates to a device comprising a processor configured to carry out the method previously described.

Another aspect of the invention relates to a computer program product comprising instructions which, when the program is executed by a computer, cause the computer to carry out the method previously described.

### BRIEF DESCRIPTION OF THE DRAWINGS

**Figure 1** illustrates a device for simulating an interaction between an endoprosthesis dedicated to be implanted in a portion of a patient aorta.
**Figure 2** is an example of a flow-chart representing a method for simulating an interaction between an endoprosthesis dedicated to be implanted in a portion of a patient aorta.
**Figure 3a and 3b** respectively illustrate a cut plane and a plurality of bidimensional cross-sections in an in-use implementation model.
**Figure 4** illustrates a distance of a point of a section of an aorta model to a point of a centerline of the aorta model in a cut plane and a distance of a point of a section of an endoprosthesis model to the point on the centerline of the aorta model in the same cut plane.
**Figure 5** is an example of a flow-chart representing a method for computing geometrical parameters used to determine at least one risk index.
**Figure 6** shows two curves representing respectively the mean aorta radius and the mean graft radius along a portion of the centerline of the aorta of a patient.
**Figure 7** is an example of an in-use implementation model where risk indices related to an oversizing of a deployed endoprosthesis are graphically represented.
**Figure 8** is an example of an in-use implementation model where risk levels related to an apposition quality of a deployed endoprosthesis are graphically represented.
**Figure 9** is an example of an in-use implementation model where risk levels related to an anchorage quality of a deployed endoprosthesis are graphically represented.
**Figures 10a and 10b** respectively illustrate an in-use implementation model corresponding to a first endoprosthesis model and to a second endoprosthesis model, where a risk of endoleak is graphically represented.

### DETAILED DESCRIPTION

This invention relates to a computer-implemented method 100 for simulating an interaction between an endoprosthesis dedicated to be implanted in a portion of a patient aorta and said portion of said patient aorta, so as to determine areas with risk of surgical complications.

Examples of surgical complications are endoleaks, endoprosthesis migration, endoprosthesis infolding, endoprosthesis occlusion, thrombosis, endoprosthesis kinks.

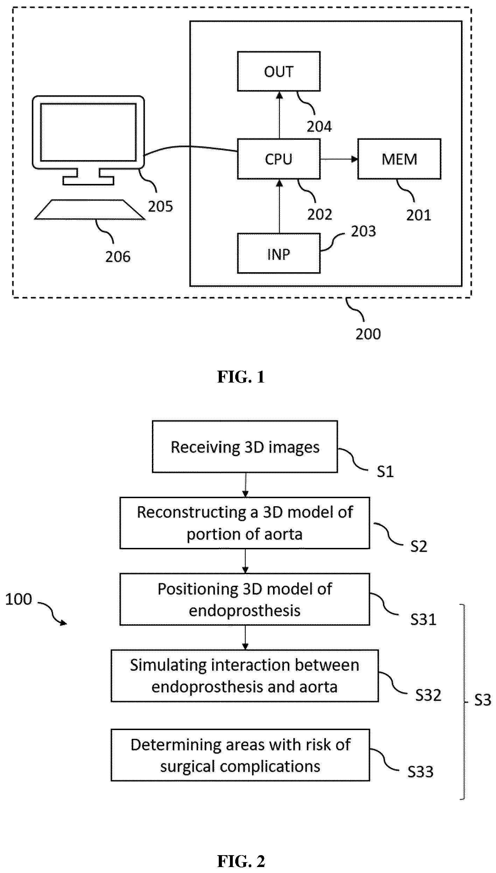

The method 100 may be implemented with a device 200 such as illustrated on Figure 1.

Figure 1 illustrates a device 200 for simulating an interaction between an endoprosthesis dedicated to be implanted in a portion of a patient aorta and said portion of said patient aorta, so as to determine areas with risk of surgical complications according to embodiments of the invention.

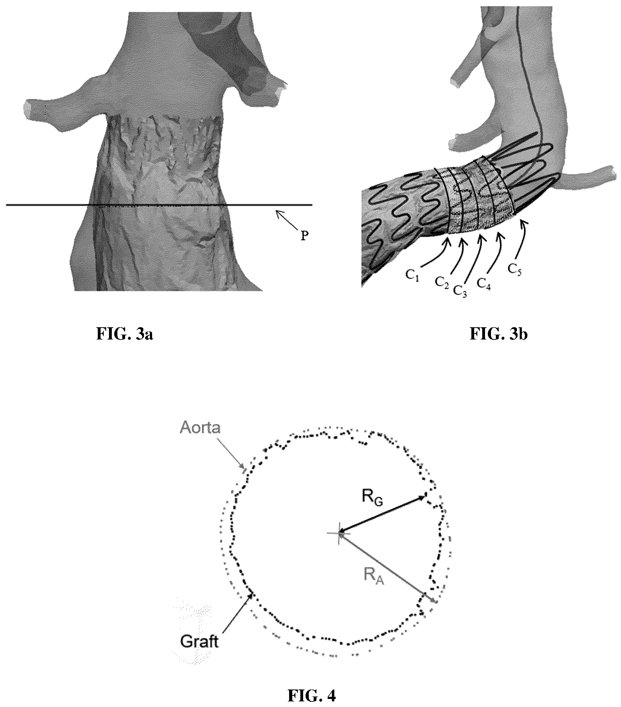

In these embodiments, the device 200 comprises a computer, this computer comprising a memory 201 to store program instructions loadable into a circuit and adapted to cause a circuit 202 to carry out steps of the methods of **Figures 2** and **3** when the program instructions are run by the circuit 202. The memory 201 may also store data and useful information for carrying steps of the present invention as described above.

The circuit 202 may be for instance:
- a processor or a processing unit adapted to interpret instructions in a computer language, the processor or the processing unit may comprise, may be associated with or be attached to a memory comprising the instructions, or
- the association of a processor / processing unit and a memory, the processor or the processing unit adapted to interpret instructions in a computer language, the memory comprising said instructions, or
- an electronic card wherein the steps of the invention are described within silicon, or
- a programmable electronic chip such as a FPGA chip (for « Field-Programmable Gate Array »).

The computer may also comprise an input interface 203 for the reception of input data and an output interface 204 to provide output data. Examples of input data and output data will be provided further.

To ease the interaction with the computer, a screen 205 and a keyboard 206 may be provided and connected to the computer circuit 202.

Figure 2 is a flowchart illustrating an example of steps to be executed to implement the computer-implemented method 100. It is supposed that an endoprosthesis will be implanted in a portion of an aorta of a patient during an intervention and that, the method 100 will determine potential risks of surgical complications based on a numerical simulation of the implantation of the endoprosthesis in the portion of the patient's aorta. The method 100 is typically implemented ahead of time of the intervention. When the endoprosthesis will be implanted in the portion of the patient's aorta, the endoprosthesis will interact with the portion of the patient's aorta. The method 100 comprises a step of a numerical simulation of this interaction, as will be described below.

In a step S1, 3D images of a portion of a patient aorta are received. For instance, the 3D images are preoperative CT scan images. The 3D images may be input through the input interface 203 of the device 200.

In a step S2, the 3D images are used to reconstruct a deformable tridimensional digital model of the portion of the patient aorta. For instance, the reconstruction is performed by the circuit 202 of the device 200. The reconstructed deformable tridimensional digital model is referred to as aorta model. For instance, when used in a numerical simulation, a technique such as described in the international patent application WO 2018/073505 A1 may be used to simulate the deformation of the aorta model.

In a step S3, a numerical simulation of the implantation of the endoprosthesis in the portion of the patient's aorta is carried out, as follows.

In a first substep S31, a tridimensional digital model of endoprosthesis is positioned at a planned location in the aorta model. The tridimensional digital model of the endoprosthesis and the aorta model form together an implantation model. For instance, the planned location corresponds to a location determined by a health practitioner responsible for the intervention.

In a second substep S32, the interaction between the endoprosthesis and the portion of the patient's aorta is simulated using the implantation model.

In a third substep S33, areas of the portion of the patient's aorta, areas with risk of surgical complications are determined based on results of said simulated interaction.

The substeps S31, S32 and S33 od step S3 may be implemented by the circuit 202 of the device 200.

According to some embodiments, in the second substep S32, the interaction is simulated using a finite element analysis method by computing contact forces between the endoprosthesis and the aorta model, then applying the computed contact forces to the tridimensional digital model of the endoprosthesis, so as to generate a deployed in-use representation of the endoprosthesis. Meanwhile, the aorta model is deformed accordingly. The generated deployed in-use representation of the endoprosthesis is referred to as deployed model. The deployed model and the deformed aorta model form together a so-called in-use implantation model.

In some embodiments, the third substep S33 may involve computing at least one risk index. For example, a global risk index is computed. In another example, a map of risk indices, where each risk index corresponds to a position in the in-use implantation model, is computed. The at least one risk index may be computed based on indicators computed from statistical data from geometrical parameters.

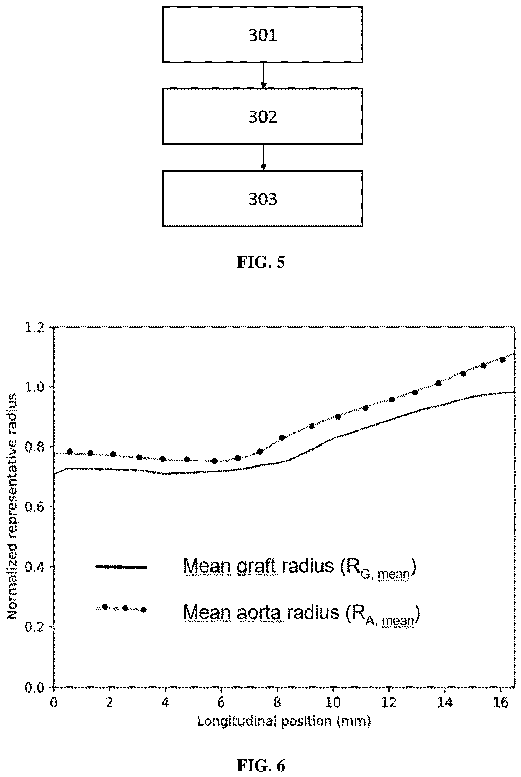

In some embodiments, the geometrical parameters are computed as follows. Bidimensional cross-sections (or slices) of the in-use implantation model are computed. A cut plane corresponds to each cross-section. Such a cut plane P is illustrated on Figure 3a. For instance, the deformed aorta model comprises a centerline. In this case, the bidimensional cross-sections can be computed along the centerline, as illustrated on Figure 3b with the curves C₁, C₂, C₃, C₄ and C₅. Those curves represent intersections of cut planes with the in-use implantation model at different points the centerline of the deformed aorta model. For instance, the deployed model comprises a stent portion and a graft portion. In a given bidimensional cross-section, a first set of points corresponds to the section of the aorta model in the corresponding cut plane, a second set of points corresponds to the section of the graft portion and a third set of points corresponds to the section of the stent portion in the corresponding cut plane. The distance of a point of the first set to the corresponding point on the centerline is denoted RAi, the distance of a point of the second set to the corresponding point on the centerline is denoted RGj and the distance of a point of the third set to the corresponding point on the centerline is denoted as RSk.. Figure 4 illustrates how the distances RAi and RGj are computed. The initial diameter of the tridimensional digital model of the endoprosthesis (not deployed) is denoted as RG0. Geometrical parameters can then be computed from the bidimensional cross-sections by implementing a computation procedure 300. An example of the computation procedure 300 is illustrated on Figure 5 and will be described below. The computation procedure 300 can for instance be implemented by the circuit 202 of the device 200, and data used during the computation procedure 300 may be stored in the memory 201 of the device 200.

In a step 301, the list of the distances RAi, RGj and RSk are stored. For instance, the list is stored in the memory 201 of the device 200.

In a step 302, the distances RAi, RGj and RSk are normalized by division by RG0, so as to obtain a list of so-called normalized aorta radii RAnormi, a list of so-called normalized graft radii RGnormj and a list of so-called normalized stent radii RSnormk.

In a step 303, statistical data are computed from the lists stored and/or obtain at steps 301 and 302. The statistical data may comprise mean, maximum, minimum or standard deviation values.

After the computation procedure 300, a list of indicators is computed, that will be used in the computation of the at least one risk index.

For instance, a list of indicators I1 to I14 that can be derived from the data stored and determined at steps 301 and 302 may be as follows:
- I1: apposition between the aorta and the endoprosthesis;
- I2: aorta, stents and graft conicity;
- I3: aorta, stents and graft straightness;
- I4: aorta, stents and graft circularity;
- I5: aorta, stents and graft diameter variability;
- I6: aorta, stents and graft stress;
- I7: pressure of the endoprosthesis on the aorta;
- I8: aorta and graft cross-section area;
- I9: aorta and graft roughness;
- I10: aorta and endoprosthesis angulation;
- I11: stents oversize;
- I12: surface of calcifications;
- I13: surface of thrombus;
- I14: patient characteristics (age, medical history).
For instance, the indicators can be computed from the data stored at step 301 and/or obtained at step 302 by the circuit 202 of the device 200.

In the present disclosure, the term "apposition" designates the contact or diameter difference between the aorta and the graft or between the aorta and the stents.

The at least one risk index may be computed from the indicators by different methods.

For instance, the at least one risk index can be computed by means of a linear or quadratic discriminant analysis. Such an analysis is a statistical analysis that classifies a data set into predefined groups. A scatter plot represents each group in an n-dimensional space, where n is the total number of indicators. The distance between each point and the barycenter of each group is used to predict whether a case belongs to a group. Decision surfaces are computed to delimit the different groups. These surfaces can be more or less complex (linear, quadratic, ...etc). This method is similar to learning techniques. Different decision surfaces in an n-dimensional space are defined during the learning process on known cases, which allows then to calculate the probability of belonging to one group or the other for a new case.

In another example, the at least one risk index can be computed by means of weighted averages. For instance, the at least one risk of index can be computed as a statistical value of several weighted averages of the indicators.

A simplified version of this latter method is the computation of a unique weighted average of all indicators.

In a typical example where the surgical complication to be determined by the method 100 is a type Ia endoleak, a relevant indicator is the indicator I1, i.e. the apposition between the aorta and the endoprosthesis. The apposition can be computed by different methods. In a first method, the sum of the difference between the graft and aorta radii can be computed. Figure 6 shows two curves representing the mean aorta radius and the mean graft radius along a portion of the centerline of the aorta. Here, the mean is calculated among all values in the cross section corresponding to a point on the centerline of the aorta. In a second method, the apposition is computed as the longest area with a lack of apposition. In a third method, the apposition is computed as the length of apposition higher than a given criterion.

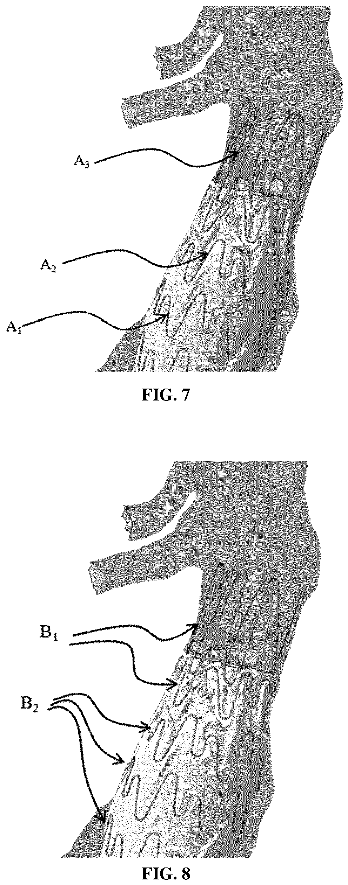

In some embodiments, the risk indices are percentages comprised between 0 and 100%, where 100% represents a high risk level. Figure 7 represents an example of in-use implantation model where areas with different risk indices, are visible. For instance, this in-use model may be visualized on the screen 205 of the device 200. In the example of Figure 7, the risk indices are related to the oversizing criterion of the deployed endoprosthesis and are determined by computing an oversizing factor, such as the indicator I11. For instance, the area A₁ corresponds to an oversizing comprised between 0 and 5 %; the area A₂ corresponds to an oversizing comprised between 5 and 10%; the area A₃ corresponds to an oversizing comprised between 20 and 25%.

In some embodiments, a risk level is attributed to areas of the in-use implantation model depending on the value of the risk index associated with the areas. For instance, when the risk index associated with an area is less than a first threshold value, the area is assigned a low risk level. When the risk index associated with an area is comprised between the first threshold value and a second threshold value, the area is assigned a medium risk level. When the risk index associated with an area is higher than the second threshold value, the area is assigned a high risk level.

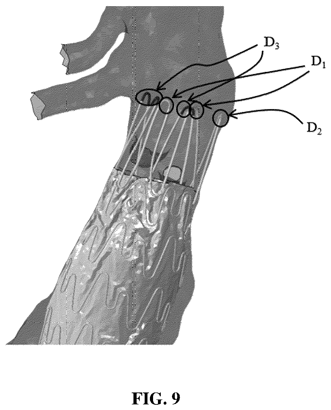

In some embodiments, the risk indices can be related to a specific characteristic pertaining to the interaction between the endoprosthesis and the portion of the patient's aorta. For instance, the risk indices can pertain to the quality of apposition of the endoprosthesis in the portion of the patient's aorta. Figure 8 illustrates an example of in-use implantation model where the level quality of apposition of several areas is can be visualized. For instance, this in-use model may be visualized on the screen 205 of the device 200. For instance, in the area B₁, the apposition of the endoprosthesis is good. In the area B₂, there is a lack of apposition. In another example, the risk indices can pertain to the quality of anchorage of the endoprosthesis in the portion of the patient's aorta. Figure 9 illustrates an example of in-use implantation model where the level of quality of anchorage of the endoprosthesis at several areas can be visualized. For instance, this in-use model may be visualized on the screen 205 of the device 200. For instance, the areas referred as D₁ present a good anchorage. The area referred as D₂ presents a low anchorage. In the areas referred as D₃, there is no anchorage.

In some embodiments, the results of the method 100 for simulating an interaction between an endoprosthesis dedicated to be implanted in a portion of a patient aorta and said portion of said patient aorta, so as to determine areas with risk of surgical complications, may be received, gathered and stored by a Web platform. The Web platform may allow visualizing those results. For instance, the Web platform allows visualizing the deployed model and the deformed aorta model, forming together a so-called in-use implantation model, with the areas with risk of surgical complications with a corresponding display pattern.

### EXAMPLES

The present invention is further illustrated by the following examples.

### Example 1: Prediction of Type Ia endoleaks after endovascular aneurysm repair (EVAR)

In this example, a retrospective cohort study was performed. The study population consisted of all patients who underwent implantation of an Endurant^{®} AAA Endovascular Graft (Medtronic, Dublin, Ireland) and presented an early or late (maximum after 5 years) type Ia endoleaks between January 2012 and September 2017 at two high-volume aortic centres. Control cases consisted of all patients who underwent implantation of the same graft without early or late type Ia endoleaks during the same period and a minimum follow-up of 5 years.

Pre-operative CT-scan were analysed with a dedicated workstation providing centerline extraction. AAA maximal diameter and proximal neck characteristics were measured using 3D and centerline reconstructions. Hostile neck anatomy was defined as one or more of the following: (1) neck length ≤15 mm, defined as the length over which the aortic diameter remains within 10% of the infra renal diameter (2) conical or tapered neck using the "neck coefficient" introducing by Albertini et al.1, (3) circumferential neck thrombus or major calcifications, (4) supra renal angulation ≥ 45 degrees and (5) infra renal angulation ≥60 degrees. Inside Endurant II/IIs 's Instructions For Use (IFU) were defined with the following neck characteristics: (1) proximal neck length of ≥10 mm, defined as the length over which the aortic diameter remains within 10% of the infra renal diameter (2) infra renal angulation of < 60°; (3) aortic neck diameters with range of 19 to 32 mm. All patients were then classified for hostile and outside the IFU criteria.

Computational technology was based on finite-element analysis and assessed the deformations induced by the device-host interaction resulting in a prediction of graft behaviour and arterial displacement. This work was based on proprietary algorithms of the applicant developed to simulate deployment of endovascular devices in patient-specific aortic aneurysms models. As the purpose of the study focused on EVAR proximal fixation, only the main graft deployment was simulated in our model.

High resolution CT-scans were used to generate patient-specific 3D models of the aorta, from the supra-renal aorta to the extern iliac arteries, including the renal arteries (aortic segmentation step) and assigned an orthotropic elastic behaviour. As calcifications of the aortic wall and intra-luminal thrombus were not modelled, the aortic wall, iliac arteries and the renal arteries were considered as homogeneous surface with a constant surface of 1.5 mm, 1 mm and 1 mm respectively. The main EndurantII/IIs^{®} device without extension was digitally reproduced on the basis of real characteristics' design of the endograft deployed in the corresponding patient. Mechanical properties of stentgraft fabric and stent rings were obtained from literature or from house mechanical testing of samples obtain from manufacturer. For the stent rings, a super-elastic material behaviour was used and the fabric was modelled as an orthotropic elastic material. Oversizing of the stent rings was respected. Deployment of the personalized stentgraft in its corresponding model of the aorta was performed using the commercially available Abaqus/Explicit 2018 finite element solver (Dassault Systèmes, Paris, France). Level of deployment was achieved in an idealized situation, just below the lowest renal artery, and blinded to the real per-operative EVAR deployment. Simulation technology assessed the deformations induced by the device-host interaction resulting in a prediction of stentgraft behaviour and arterial displacement. The methodology implied a step called "morphing", which is presented in the method described in the international patent application WO 2018/073505 A1, during which the aortic surface was numerically deformed until a cylindric shape is obtained, allowing deployment of a stentgraft inside. Then a reverse deformation of the aortic aorta was achieved while maintaining the stentgraft in place.

Mean oversizing and stentgraft apposition assessment on the two first covered stents of the main stentgraft were measured on simulation results.

Aortic and graft sections were made every 0.5 mm at the level of the first two stents of the covered part of the stentgraft. For each section, a normalized graft and aorta representative radii were calculated in order to avoid systematic bias relative to diameter.

"Aspect ratio" was evaluated by the difference between the maximal and minimal aortic normalized radii, and the difference between the maximal and minimal graft normalized radii after deployment simulation.

Apposition was evaluated by the difference of the area under the curve of the aortic representative radii and the area under the curve of the graft representative radii.

A risk index was calculated by the method 100 previously described, based on the aspect ratio indicator and on the apposition factor. A quadratic discriminant analysis has been used to classify the cases in two groups: with or without risk of type Ia endoleak.

Normality could not be assumed for a large part of data because of small numbers. Thus, descriptive data were displayed as medians with interquartile ranges (Q1, Q3). Statistical differences in indexes were assessed with the non-parametric Mann Whitney U test for continuous variables and χ2 or the Fischer's exact test for categorical variables. Statistical significance was assumed at p <0.05; all reported p -values were two-sided. All statistical analysis were performed with the R statistical software version 4.2.2 (A language and environment for statistical computing. R foundation for Statistical Computing, Vienna, Austria).

### Results

162 cases were analysed and 15 type Ia endoleaks cases and 14 control cases with sufficient follow-up were identified. Mean follow up was 1.9 years [0-8.7] in the type Ia endoleaks group and 6.8 years [5.2-10.3] in the control group. In the type Ia endoleak group, 8 cases (53.3%) presented a per-operative or early type Ia endoleak and 7 (46,7%) late type Ia endoleaks (from 8 months to 5 years). Table 2 shows, for the 15 type Ia endoleaks cases and 14 control cases, the comparison between the real observation of type Ia endoleaks and the prediction with the method 100. The filled circles represent a presence of a type Ia endoleak, while the crosses represent an absence of a type Ia endoleak.

| Case number | TYPE Ia ENDOLEAK | | Endoleak delay/Last follow-up |
|---|---|---|---|
| | Real Observation | Simulation with the method 100 | |
| 1 | • | • | Peroperative |
| 2 | • | • | 5 years |
| 3 | • | • | Peroperative |
| 4 | • | • | Peroperative |
| 5 | • | • | Peroperative |
| 6 | • | • | Peroperative |
| 7 | • | • | 1 day |
| 8 | • | • | 5 years |
| 9 | • | • | Peroperative |
| 10 | • | • | Peroperative |
| 11 | • | • | 2 years |
| 12 | • | • | 5 years |
| 13 | • | • | 3 years |
| 14 | • | • | 8 months |
| 15 | • | • | 2 years |
| 16 | X | X | 6 years |
| 17 | X | X | 7 years |
| 18 | X | X | 9 years |
| 19 | X | X | 5 years |
| 20 | X | X | 7 years |
| 21 | X | X | 7 years |
| 22 | X | X | 5 years |
| 23 | X | X | 5 years |
| 24 | X | X | 5 years |
| 25 | X | X | 10 years |
| 26 | X | X | 8 years |
| 27 | X | X | 8 years |
| 28 | X | X | 9 years |
| 29 | X | X | 7 years |

### Example 2: Assistance in the choice of endoprosthesis during the planification stage

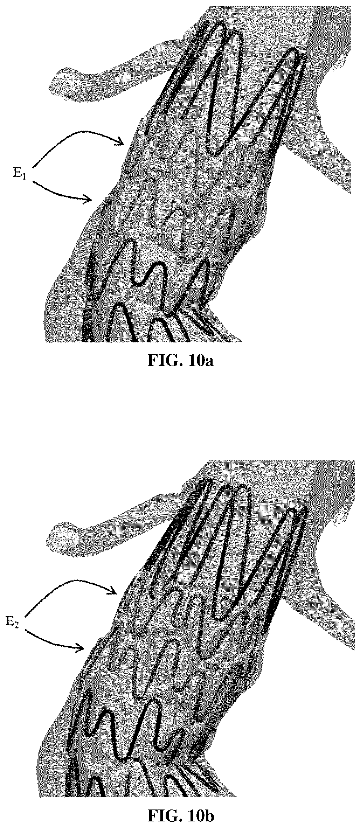

By determining areas with risk of surgical complications, the method 100 can be of help to choose a suitable endoprosthesis that will minimize the risk of surgical complication. Figure 10a represents an example of in-use implantation model where a model of an Endurant II device of type ETBF2516C166EE, a standard size device, is implanted inside a portion of the aorta of a patient. The area E₁ in the in-use implantation model has been determined as presenting a high risk of endoleak. Figure 10b represents a second example of in-use implantation model where a model of another Endurant II device, of type ETBF2816C166EE, is implanted inside the portion of the aorta of the same patient. The area E2 has been determined, with this other device, as presenting a low risk of endoleak.

## Claims

1. A computer-implemented method (100) for simulating an interaction between an endoprosthesis dedicated to be implanted in a portion of a patient aorta and said portion of said patient aorta, so as to determine areas with risk of surgical complications, comprising the steps of:
- receiving (S1) 3D images of said portion of the patient aorta,
- reconstructing (S2) a deformable tridimensional digital model of said portion of the patient aorta based on said 3D images, the reconstructed deformable tridimensional digital model being referred to as aorta model,
- positioning (S31) a tridimensional digital model of said endoprosthesis at a planned location in the aorta model, said tridimensional model of said endoprosthesis and said aorta model forming together an implantation model,
- simulating (S32) said interaction based on a computation using said implantation model, **characterized by**
- determining (S33) based on results of said simulated interaction, at least one risk index of surgical complications for predefined areas of the implantation model, said at least one risk index being computed based on indicators obtained from statistical data from geometrical parameters, so to obtain a map of risk indices, where each risk index corresponds to a position in said implantation model.

2. The computer-implemented method (100) according to claim 1, wherein the surgical complications comprise at least one among endoleaks, endoprosthesis migration, endoprosthesis infolding, endoprosthesis occlusion, thrombosis, and endoprosthesis kinks.

3. The computer-implemented method (100) according to any one of claims 1 or 2, wherein the computation involving said implantation model comprises a computation of a geometrical deformation of said tridimensional digital model of said endoprosthesis inside the aorta model.

4. The computer-implemented method (100) according to claim 3, wherein the computation of the geometrical deformation is performed by finite element analysis.

5. The computer-implemented method (100) according to any of the preceding claims, wherein said 3D images are preoperative CT scan images.

6. The computer-implemented method (100) according to any of the preceding claims, wherein the interaction is an endovascular repair of aortic aneurysms.

7. The computer-implemented method (100) according to any of the preceding claims, further comprising, after determining the areas with risk of surgical complication, a step of determining, for each area with risk of surgical complication, a risk level.

8. The computer-implemented method (100) according to claim 7, wherein the step of determining a risk level comprises a computation involving an apposition index or an aspect ratio.

9. The computer-implemented method (100) according to claim 7 or 8, wherein each risk level is associated with a corresponding display pattern, the method further comprising a step of applying the corresponding display pattern to each area with risk of surgical complication.

10. The computer-implemented method (100) according to claim 9, further comprising a step of displaying the implantation model on a visualization unit with the areas with risk of surgical complication with the applied corresponding display pattern.

11. Use of the computer-implemented method (100) according to any of the preceding claims for planning an abdominal aortic aneurysm repair surgery using areas with risk of surgical complications determined by the method according to any of the preceding claims.

12. A device comprising a processor configured to carry out a method according to any one of claims 1 to 10.

13. A computer program product comprising instructions which, when the program is executed by a computer, cause the computer to carry out the method of any one of claims 1 to 10.

## Patentansprüche

1. Computerimplementiertes Verfahren (100) zum Simulieren einer Wechselwirkung zwischen einer Endoprothese, welche dazu bestimmt ist, in einen Abschnitt der Aorta eines Patienten implantiert zu werden, und dem Abschnitt der Aorta des Patienten, um Bereiche mit einem Risiko für chirurgische Komplikationen zu bestimmen, umfassend die folgenden Schritte:
- Empfangen (S1) von 3D-Bildern des betreffenden Abschnitts der Aorta des Patienten,
- Rekonstruieren (S2) eines deformierbaren dreidimensionalen digitalen Modells des Abschnitts der Aorta des Patientena basierend auf 3D-Bildern wobei das rekonstruierte deformierbare dreidimensionale digitale Modell als Aortenmodell bezeichnet wird,
- Positionieren (S31) eines dreidimensionalen digitalen Modells der Endoprothese an einer geplanten Stelle in dem Aortenmodell, wobei das dreidimensionale Modell der Endoprothese und das Aortenmodell zusammen ein Implantationsmodell bilden,
- Simulieren (S32) der Interaktion basierend auf einer Berechnung unter Verwendung des Implantationsmodells, **gekennzeichnet durch**:
- Bestimmen (S33) mindestens eines Risikoindex für chirurgische Komplikationen für vordefinierte Bereiche des Implantationsmodells basierend auf den Ergebnissen der simulierten Wechselwirkung, wobei mindestens ein Risikoindex basierend auf Indikatoren berechnet wird, welche aus statistischen Daten von geometrischen Parametern gewonnen werden, um eine Karte von Risikoindizes zu erhalten, wobei jeder Risikoindex einer Position in dem Implantationsmodell entspricht.

2. Computerimplementiertes Verfahren (100) nach Anspruch 1, wobei die chirurgischen Komplikationen mindestens eine aus Endoleaks, Endoprothesenmigration, Endoprothesenfaltung, Endoprothesenokklusion, Thrombose und Endoprothesenknicken umfassen.

3. Computerimplementiertes Verfahren (100) nach einem der Ansprüche 1 oder 2, wobei die Berechnung des Implantationsmodells eine Berechnung einer geometrischen Verformung des dreidimensionalen digitalen Modells der Endoprothese innerhalb des Aortenmodells umfasst.

4. Computerimplementiertes Verfahren (100) nach Anspruch 3, wobei die Berechnung der geometrischen Verformung durch Finite-Elemente-Analyse durchgeführt wird.

5. Computerimplementiertes Verfahren (100) nach einem der vorstehenden Ansprüche, wobei es sich bei den 3D-Bildern um präoperative Bilder eines CT-Scans handelt.

6. Computerimplementiertes Verfahren (100) nach einem der vorstehenden Ansprüche, wobei die Wechselwirkung eine endovaskuläre Reparatur von Aortenaneurysmen ist.

7. Computerimplementiertes Verfahren (100) nach einem der vorstehenden Ansprüche, welches nach Bestimmen der Bereiche mit Risiko für chirurgische Komplikationen weiter einen Schritt des Bestimmens eines Risikoniveaus für jeden Bereich mit Risiko für chirurgische Komplikationen umfasst.

8. Computerimplementiertes Verfahren (100) nach Anspruch 7, wobei der Schritt des Bestimmens eines Risikoniveaus eine Berechnung umfasst, welche einen Appositionsindex oder ein Seitenverhältnis umfasst.

9. Computerimplementiertes Verfahren (100) nach Anspruch 7 oder 8, wobei jedes Risikoniveau mit einem entsprechenden Anzeigemuster verknüpft ist, wobei das Verfahren weiter einen Schritt des Anwendens des entsprechenden Anzeigemusters auf jeden Bereich mit Risiko für chirurgische Komplikation umfasst.

10. Computerimplementiertes Verfahren (100) nach Anspruch 9, welches weiter einen Schritt des Anzeigens des Implantationsmodells auf einer Visualisierungseinheit mit den Bereichen mit Risiko für chirurgische Komplikationen mit dem angewendeten entsprechenden Anzeigemuster umfasst.

11. Verwendung des computerimplementierten Verfahrens (100) nach einem der vorstehenden Ansprüche zum Planen einer Operation zur Reparatur eines abdominalen Aortenaneurysmas unter Verwendung von Bereichen mit Risiko für chirurgische Komplikationen, welche durch das Verfahren nach einem der vorstehenden Ansprüche bestimmt werden.

12. Vorrichtung, umfassend einen Prozessor, welcher dazu konfiguriert ist, ein Verfahren nach einem der Ansprüche 1 bis 10 durchzuführen.

13. Computerprogrammprodukt, umfassend Anweisungen, welche, wenn das Programm von einem Computer ausgeführt wird, den Computer dazu veranlassen, die Schritte des Verfahrens nach einem der Ansprüche 1 bis 10 auszuführen.

## Revendications

1. Un procédé mis en œuvre par ordinateur (100) pour simuler une interaction entre une endoprothèse destinée à être implantée dans une partie de l'aorte d'un patient et ladite partie de l'aorte dudit patient, afin de déterminer des zones présentant un risque de complications chirurgicales, le procédé comprenant les étapes consistant à :
- recevoir (S1) des images 3D de ladite partie de l'aorte du patient ;
- reconstruire (S2) un modèle numérique tridimensionnel déformable de ladite partie de l'aorte du patient sur la base desdites images 3D, le modèle numérique tridimensionnel déformable reconstruit étant désigné comme un modèle d'aorte ;
- positionner (S31) un modèle numérique tridimensionnel de ladite endoprothèse à un emplacement planifié dans le modèle d'aorte, ledit modèle tridimensionnel de ladite endoprothèse et ledit modèle d'aorte formant ensemble un modèle d'implantation ;
- simuler (S32) ladite interaction sur la base d'un calcul utilisant ledit modèle d'implantation ; **caractérisé par**
- déterminer (S33), sur la base des résultats de ladite interaction simulée, au moins un indice de risque de complications chirurgicales pour des zones prédéfinies du modèle d'implantation, ledit au moins un indice de risque étant calculé sur la base d'indicateurs obtenus à partir de données statistiques de paramètres géométriques, de manière à obtenir une carte d'indices de risque, dans laquelle chaque indice de risque correspond à une position dans ledit modèle d'implantation.

2. Le procédé mis en œuvre par ordinateur (100) selon la revendication 1, dans lequel les complications chirurgicales comprennent au moins l'une parmi des endofuites, une migration de l'endoprothèse, un repliement de l'endoprothèse, une occlusion de l'endoprothèse, une thrombose et des coudes de l'endoprothèse.

3. Le procédé mis en œuvre par ordinateur (100) selon l'une quelconque des revendications 1 ou 2, dans lequel le calcul mettant en œuvre ledit modèle d'implantation comprend un calcul d'une déformation géométrique dudit modèle numérique tridimensionnel de ladite endoprothèse à l'intérieur du modèle d'aorte.

4. Le procédé mis en œuvre par ordinateur (100) selon la revendication 3, dans lequel le calcul de la déformation géométrique est effectué par analyse par éléments finis.

5. Le procédé mis en œuvre par ordinateur (100) selon l'une quelconque des revendications précédentes, dans lequel lesdites images 3D sont des images de scanner tomodensitométrique préopératoire.

6. Le procédé mis en œuvre par ordinateur (100) selon l'une quelconque des revendications précédentes, dans lequel l'interaction est une réparation endovasculaire d'anévrisme aortique.

7. Le procédé mis en œuvre par ordinateur (100) selon l'une quelconque des revendications précédentes, comprenant en outre, après la détermination des zones présentant un risque de complication chirurgicale, une étape consistant à déterminer, pour chaque zone présentant un risque de complication chirurgicale, un niveau de risque.

8. Le procédé mis en œuvre par ordinateur (100) selon la revendication 7, dans lequel l'étape de détermination d'un niveau de risque comprend un calcul mettant en œuvre un indice d'apposition ou un rapport d'aspect.

9. Le procédé mis en œuvre par ordinateur (100) selon la revendication 7 ou 8, dans lequel chaque niveau de risque est associé à un motif d'affichage correspondant, le procédé comprenant en outre une étape consistant à appliquer le motif d'affichage correspondant à chaque zone présentant un risque de complication chirurgicale.

10. Le procédé mis en œuvre par ordinateur (100) selon la revendication 9, comprenant en outre une étape consistant à afficher le modèle d'implantation sur une unité de visualisation, avec les zones présentant un risque de complication chirurgicale auxquelles est appliqué le motif d'affichage correspondant.

11. Le procédé de planification d'une intervention de réparation d'un anévrisme de l'aorte abdominale utilisant des zones présentant un risque de complications chirurgicales déterminées par le procédé selon l'une quelconque des revendications précédentes.

12. Un dispositif comprenant un processeur configuré pour mettre en œuvre le procédé selon l'une quelconque des revendications 1 à 10.

13. Un produit programme d'ordinateur comprenant des instructions qui, lorsque le programme est exécuté par un ordinateur, amènent celui-ci à mettre en œuvre le procédé selon l'une quelconque des revendications 1 à 10.
